Europäisches Patentamt

European Patent Office

Office européen des brevets

(11) Publication number : **0 581 587 A2**

(12) # EUROPEAN PATENT APPLICATION

(21) Application number : 93305970.1

(51) Int. Cl.⁵ : **A61K 9/06,** A61M 35/00

(22) Date of filing : 28.07.93

(30) Priority : 31.07.92 JP 204587/92
16.03.93 JP 55245/93

(43) Date of publication of application :
02.02.94 Bulletin 94/05

(84) Designated Contracting States :
AT BE CH DE DK ES FR GB GR IE IT LI LU MC NL PT SE

(71) Applicant : TANABE SEIYAKU CO., LTD.
2-10, Dosho-machi 3-chome
Chuo-ku Osaka (JP)

(72) Inventor : Kobayashi, Masao
No. 1, Shimokawara-cho, Nanzenji, Sakyo-ku
Kyoto-shi, Kyoto-fu (JP)
Inventor : Suzuki, Takehiko
No.9-20, Koufudai 5-chome, Toyono-cho
Toyono-gun, Osaka-fu (JP)
Inventor : Sugaya, Kayo
Itami Park-Homes 3-601
No.110-1,Kitahonmachi
1-chome Itami-shi,Hyogo-ken (JP)
Inventor : Harada, Mitsunori
No.12-10-1014, Kitahorie 4-chome Nishi-ku
Osaka-shi, Osaka-fu (JP)

(74) Representative : Hardisty, David Robert et al
BOULT, WADE & TENNANT 27 Furnival Street
London EC4A IPQ (GB)

(54) Base for transdermal administration.

(57) A base for transdermal administration comprising a fatty acid ester, an alcohol and water and a pharmaceutical preparation for transdermal administration comprising a medicinal compound and the base. According to the present invention, even a medicinal compound which has been difficult to be absorbed transdermally, can be efficiently administered transdermally.

EP 0 581 587 A2

Jouve, 18, rue Saint-Denis, 75001 PARIS

The present invention relates to a novel base for transdermal administration and a novel pharmaceutical preparation for transdermal administration comprising the base and a medicinal compound.

When a medicinal compound is administered orally, there are many problems in some cases such as low bioavailability owing to first pass effect in liver, degradation in gastrointestinal tructs, low absorption through gastrointestinal membrane, and so on. Furthermore, it is sometimes difficult to orally administer to an aged person or a bed-ridden patient. Recently transdermal administration has therefore been given attention, because there are advantages that it is easy to administrate, bring about prolonged effect and lower side effect.

There has been, however, a problem that a medicinal compound penetrates through the stratum corneum slowly because the stratum corneum in epidermis acts as a barrier against absorption of the medicinal compound. Further, dermis may also acts as a barrier against the penetration of a medicinal compound with high lipophilicity.

In order to solve the above-mentioned problem, there have been used as a penetration enhancer, Azone (chemical name: 1-dodecylazacycloheptan-2-one), menthol, alkylmethylsulfoxides, pyrrolidone or the like. With respect to a base for transdermal administration, there have also been known base components having penetration-enhancing ability, for example, alcohols, fatty acids, fatty acid esters and the like.

Furthermore, there have also been known pharmaceutical preparations for transdermal administration bringing about improved absorption property of a medicinal compound. Such preparation is prepared by combining a specific base and a specific medicinal compound, as described in Japanese Unexamined Patent Publication No. 152921/1990 which discloses a pharmaceutical preparation for transdermal administration comprising a β-blocker such as propranolol hydrochloride and a base composed of (a) a higher alcohol such as lauryl alcohol or oleyl alcohol, (b) a higher fatty acid such as oleic acid or lauric acid and (c) at least one of ethanol, isopropyl alcohol and water.

However, there are restrictions in practical use of the above-mentioned transdermal penetration enhancers, because the enhancer may cause strong skin irritation or has a problem in safety. Furthermore, even in case that the above-mentioned known base is used in order to improve the transdermal absorption of a medicinal compound, an effective result is not always achieved with all compounds, but achieved only with specific compounds. There has not been known a base which shows the transdermal penetration enhancement effect for any medicinal compounds.

An object of this invention is to provide a base which shows the transdermal penetration enhancement effect for many medicinal compounds.

It has been found that a base for transdermal administration comprising a fatty acid ester, an alcohol and water enables even a medicinal compound, which has been hitherto relatively difficult to be transdermally administered to penetrate skin epidermis and dermis with a high penetration ratio without using any penetration enhancer, and shorten the lag time in the absorption of a medicinal compound. It has also been found that the above-mentioned effects are exhibited only by a base comprising the above-mentioned three components, and moreover the above-mentioned base can be used for many medicinal compounds. Further, the above-mentioned base has very little skin irritation and therefore a pharmaceutical preparation comprising the base and a medicinal compound has excellent safety.

Although the mechanism of such excellent penetration enhancement effect in the present invention is not fully clear, it can be understood as follows:

A fatty acid ester is mainly distributed to the stratum corneum because of its lipophilicity and interacts with the stratum corneum lipid. An alcohol dissolves both of a hydrophilic medicinal compound and a lipophilic medicinal compound therein and serves as a carrier of the medicinal compound for penetrating a skin. Particularly, a lower monohydric alcohol, such as ethanol, also has a function to increase the stratum corneum liquid fluidity or a function to extract lipids from the stratum corneum. Water serves to increase the solubility of a hydrophilic medicinal compound in the base and serves to accelerate a release of a lipophilic medicinal compound from the base, in addition to a skin hydration. It is considered that a synergistic penetration enhancing effect can be achieved by combining the above-mentioned three components.

Accordingly the invention provides a base comprising a fatty acid ester, an alcohol and water, and a pharmaceutical preparation for transdermal administration comprising the base and a medicinal compound.

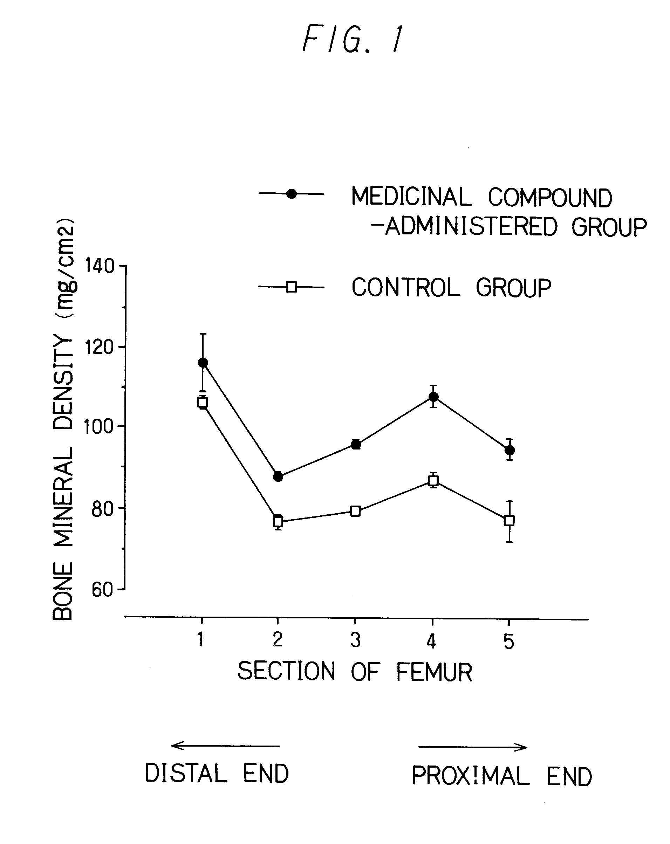

The invention is illustrated by Figure 1 which shows the bone mineral density of every section of femur of a vitamin D deficient rat.

In the present invention, as the fatty acid ester, there may be used (A) an ester of a fatty acid having 1 to 32 carbon atoms and an aliphatic monohydric alcohol having 1 to 30 carbon atoms, (B) an ester of a saturated or unsaturated fatty acid having 10 to 22 carbon atoms and a mono- or polyglycerin and the like.

Representative examples of (A) are, for instance, an ester of fatty acid such as formic acid, acetic acid, propionic acid, butyric acid, valeric acid, caproic acid, enanthic acid, caprylic acid, pelargonic acid, capric acid, undecylic acid, lauric acid, tridecylic acid, myristic acid, pentadecylic acid, palmitic acid, heptadecylic acid, stearic acid, oleic acid, nonadecanoic acid, arachic acid, obtusilic acid, linoleic acid, linolenic acid, linderic acid, arachidonic acid, sebacic acid, behenic acid, lignoceric acid, cerotic acid, heptacosanoic acid, montanic acid, melissic acid, lacceric acid, elaidic acid or brassidic acid; and an aliphatic monohydric alcohol such as methyl alcohol, ethyl alcohol, propyl alcohol, isopropyl alcohol, butyl alcohol, isobutyl alcohol, sec-butyl alcohol, tert-butyl alcohol, n-amyl alcohol, isoamyl alcohol, hexyl alcohol, heptyl alcohol, octyl alcohol, capryl alcohol, nonyl alcohol, decyl alcohol, undecyl alcohol, lauryl alcohol, tridecyl alcohol, myristyl alcohol, pentadecyl alcohol, cetyl alcohol, isocetyl alcohol, hexadecyl alcohol, heptadecyl alcohol, stearyl alcohol, oleyl alcohol, octyldodecyl alcohol, nonadecyl alcohol, eicosyl alcohol, ceryl alcohol or melissyl alcohol, and the like.

As a preferable fatty acid ester among these, there can be exemplified an ester of a fatty acid having 6 to 32, particularly 6 to 18 carbon atoms, such as caproic acid, enanthic acid, caprylic acid, pelargonic acid, capric acid, undecylic acid, lauric acid, tridecylic acid, myristic acid, pentadecylic acid, palmitic acid, heptadecylic acid, stearic acid, oleic acid, nonadecanoic acid, arachic acid, obtusilic acid, linoleic acid, linolenic acid, linderic acid, arachidonic acid, sebacic acid, behenic acid, lignoceric acid, cerotic acid, heptacosanoic acid, montanic acid, melissic acid, lacceric acid, elaidic acid or brassidic acid; and an aliphatic monohydric alcohol having 1 to 20, particularly 3 to 20 carbon atoms, such as methyl alcohol, ethyl alcohol, propyl alcohol, isopropyl alcohol, butyl alcohol, isobutyl alcohol, sec-butyl alcohol, tert-butyl alcohol, n-amyl alcohol, isoamyl alcohol, cetyl alcohol, isocetyl alcohol, stearyl alcohol or octyldodecyl alcohol, and the like.

Specific examples of the fatty acid ester (A), include, for example, methyl caprate, ethyl caprate, isopropyl caprate, butyl caprate, methyl caproate, ethyl caproate, isopropyl caporate, butyl caproate, methyl palmitate, ethyl palmitate, butyl palmitate, isopropyl palmitate, isostearyl palmitate, methyl myristate, ethyl myristate, butyl myristate, isopropyl myristate, isocetyl myristate octyldodecyl myristate, butyl stearate, isocetyl isostearate, ethyl linolate, isopropyl linolate, butyl linolate and the like.

Among these, there are preferable isopropyl caproate, isopropyl caprate, isopropyl palmitate, isopropyl myristate, butyl palmitate, butyl myristate, octyldodecyl myristate, butyl stearate, isocetyl isostearate, isopropyl linolate and butyl linolate, particularly, isopropyl caproate, isopropyl caprate, isopropyl myristate, isocetyl isostearate, butyl stearate, isopropyl palmitate and octyldodecyl myristate.

Representative examples of (B) are, for instance, an ester of a saturated or unsaturated fatty acid having 10 to 22, particularly 12 to 18 carbon atoms, such as capric acid, undecylic acid, lauric acid, tridecylic acid, myristic acid, pentadecylic acid, palmitic acid, heptadecylic acid, stearic acid, oleic acid, nonadecanoic acid, arachic acid, linoleic acid, linolenic acid or behenic acid; and monoglycerin or polyglycerin, particularly monoglycerin, diglycerin, triglycerin, tetraglycerin, pentaglycerin or hexaglycerin, and the like.

Specific examples of the fatty acid ester (B), include, for example, glyceryl monolinolate, glyceryl monoisostearate, glyceryl monolaurate, glyceryl monomyristate, glyceryl monostearate, glyceryl monooleate, glyceryl dilaurate, glyceryl dimyristate, glyceryl distearate, glyceryl trilaurate, glyceryl trimyristate, glyceryl tristearate, diglyceryl monostearate, diglyceryl monooleate, diglyceryl monoisostearate, diglyceryl dioleate, diglyceryl monolaurate, hexaglyceryl monolaurate, hexaglyceryl monomyristate, hexaglyceryl monostearate, hexaglyceryl monooleate, decaglyceryl monomyristate, decaglyceryl monostearate, decaglyceryl monooleate, decaglyceryl monolinolate and the like.

Among these, there are preferable glyceryl monooleate, glyceryl dioleate, glyceryl isostearate, hexaglyceryl monolaurate and hexaglyceryl monomyristate, particularly, hexaglyceryl monolaurate and glyceryl monooleate.

As the alcohol which is a component of the base of the present invention, a monohydric or polyhydric alcohol having 2 to 12 carbon atoms can be exemplified.

Representative examples of the monohydric alcohol are, for instance, a saturated or unsaturated aliphatic monohydric alcohol such as ethyl alcohol, propyl alcohol, isopropyl alcohol, butyl alcohol, isobutyl alcohol, sec-butyl alcohol, tert-butyl alcohol, n-amyl alcohol, isoamyl alcohol, hexyl alcohol, heptyl alcohol, octyl alcohol, capryl alcohol, nonyl alcohol, decyl alcohol, undecyl alcohol or lauryl alcohol and the like.

Among these, there are preferable an aliphatic alcohol having 2 to 10 carbon atoms, such as ethyl alcohol, propyl alcohol, isopropyl alcohol, butyl alcohol, isobutyl alcohol, sec-butyl alcohol, tert-butyl alcohol, n-amyl alcohol, isoamyl alcohol, hexyl alcohol, heptyl alcohol or octyl alcohol and the like. Further, there are more preferable an aliphatic alcohol having 2 to 5 carbon atoms, such as ethyl alcohol, propyl alcohol, isopropyl alcohol, butyl alcohol, isobutyl alcohol, sec-butyl alcohol, tert-butyl alcohol, n-amyl alcohol or isoamyl alcohol, particularly, ethyl alcohol, isopropyl alcohol and butyl alcohol.

Representative examples of the polyhydric alcohol are, for instance, a saturated or unsaturated aliphatic polyhydric alcohol such as an aliphatic dihydric alcohol, e.g. ethylene glycol, propylene glycol, trimethylene glycol, glycerin$\alpha$-monochlorohydrin, 1,2-butanediol, 1,3-butanediol, 2,3-butanediol, 1,4-butanediol, isobutylene glycol, pentamethylene glycol, trimethylethylene glycol, hexamethylene glycol, 2,5-hexanediol, 2-methyl-2,4-pentadiol, butanediol or 2-butin-1,4-diol; an aliphatic polyhydric alcohol, e.g. glycerin, and the like.

Among these, there are preferable ethylene glycol, propylene glycol, 1,2-butanediol, 1,3-butanediol and glycerin, particularly, propylene glycol, 1,3-butanediol and glycerin.

That is, a monohydric, dihydric or trihydric alcohol having 2 to 4 carbon atoms is most preferable as the alcohol.

As water which is another component of the base of the present invention, water having properties usually usable in the field of pharmaceutical preparation can be suitably used. A salt, a saccharide, a polymer and an acid or an alkali for controlling pH can be further added to water according to the property of the medicinal compound.

An amount of each component in the base of the present invention can be suitably determined according to a kind of a medicinal compound to be used, and there is no particular difficulty for a person skilled in the field of pharmaceutical preparation to find a suitable range of the amount.

For instance, the suitable range can be determined with taking aim at a composition wherein the three components are miscible or almost miscible with each other so that the three components do not separate from each other when mixing them.

The miscibility sometimes changes depending on the number of carbon atoms of the fatty acid ester or the alcohol to be mixed in the base and/or depending on an additive such as acid or alkali in water. Therefore alternatively, even in case of not taking aim at the miscibility, a desired base composition can be easily determined by evaluating, according to a usual method, a skin permeability of a medicinal compound contained in each base composition, with suitably changing the proportion of the amounts of the three components in the composition.

In a preferable embodiment of the present invention, the base comtains about 0.1 to about 50 % by volume of the fatty acid ester, about 10 to about 90 % by volume of the alcohol and about 1 to about 80 % by volume of water on the basis of the total volume of the three components. More preferably, the base contains about 1 to about 30 % by volume of the fatty acid ester, about 25 to about 80 % by volume of the alcohol and about 1 to about 50 % by volume of water on the basis of the total volume of the three components.

The proportions of the components in the base are explained below in connection with the number of carbon atoms of the alcohol.

i ) When a monohydric or polyhydric alcohol having at most 4 carbon atoms is used as a component of the base, the alcohol is used in an amount of about 10 to about 90 % by volume on the basis of the total volume of the three components, and water and the fatty acid ester can be used within the above ranges.

In this case, when as the fatty acid ester an ester of a fatty acid having about 10 to about 32 carbon atoms and an aliphatic monohydric alcohol is used, the ester which is in a liquid state at normal temperature is suitably used in an amount of about 0.1 to about 50 % by volume, and the ester which is in a solid state at normal temperature is suitably used in an amount of about 0.1 to about 30 % by volume, respectively together with about 1 to about 80 % by volume of water, on the basis of total volume of the three components.

When as the fatty acid ester an ester of a saturated or unsaturated fatty acid having about 10 to about 22 carbon atoms and monoglycerin or a polyglycerin is used, about 5 to about 50 % by volume of the fatty acid ester which has HLB (hydrophile-lipophile balance) of at least 9.0 and about 10 to about 80 % by volume of water are suitably used and about 0.1 to about 30 % by volume of the fatty acid ester which has HLB of less than 9.0 and about 1 to about 40 % by volume of water are suitably used, on the basis of total volume of the three components.

ii ) When a monohydric or polyhydric alcohol having at least 5 carbon atoms is used as a component of the base, the alcohol is used in an amount of about 10 to about 90 % by volume on the basis of the total volume of the three components, and water and the ester can be used within the above ranges.

In this case, when as the fatty acid ester an ester of a fatty acid having about 10 to about 32 carbon atoms and an aliphatic monohydric alcohol is used, the ester which is in a liquid state at normal temperature is suitably used in an amount of about 0.1 to about 30 % by volume, and the ester which is in a solid state at normal temperature is suitably used in an amount of about 0.1 to about 20 % by volume, respectively together with about 1 to about 80 % by volume of water, on the basis of the total volume of the three components.

When as the fatty acid ester an ester of a saturated or unsaturated fatty acid having about 10 to about 22 carbon atoms and monoglycerin or a polyglycerin is used, about 5 to about 50 % by volume of the fatty acid ester which has HLB of at least 9.0 and about 10 to about 80 % by volume of water are suitably used, and about 0.1 to about 30 % by volume of the fatty acid ester which has HLB of less than 9.0 and about 1 to about 40 % by volume of water are suitably used, on the basis of total volume of the three components.

Though all of the above-mentioned combinations are suitable themselves, a base using as the alcohol a monohydric or polyhydric alcohol having at most 4 carbon atoms is rather preferable. Furthermore, it is more preferable to use a base using an ester, which is in a liquid state at normal temperature, of a fatty acid having about 10 to about 32 carbon atoms and an aliphatic monohydric alcohol, or an ester, whose HLB is at least 9.0, of a saturated or unsaturated fatty acid having 10 to 22 carbon atoms and monoglycerin or a polyglycerin, together with a monohydric or polyhydric alcohol having at most 4 carbon atoms.

With respect to concrete combination of the components for the base of the present invention, there can be exemplified following bases having preferable proportion of the components. In case of a base comprising isopropyl myristate, ethyl alcohol and water, about 1 to about 30 % by volume of isopropyl myristate, about 25 to about 80 % by volume of ethyl alcohol and about 5 to about 60 % by volume of water are used on the basis of the total volume of the three components. In case of a base comprising isopropyl myristate, isopropyl alcohol and water, about 1 to about 50 % by volume of isopropyl myristate, about 25 to about 75 % by volume of isopropyl alcohol and about 5 to about 60 % by volume of water are used on the basis of the total volume of the three components. In case of a base comprising isopropyl myristate, tert-butyl alcohol and water, about 1 to about 30 % by volume of isopropyl myristate, about 10 to about 70 % by volume of tert-butyl alcohol and about 5 to about 80 % by volume of water are used on the basis of the total volume of the three components. In the case of a base comprising isopropyl myristate, butyl alcohol and water, about 1 to about 40 % by volume of isopropyl myristate, about 10 to about 75 % by volume of butyl alcohol and about 5 to about 80 % by volume of water are used on the basis of the total volume of the three components.

The above-mentioned base of the present invention has not only suitable moisturizing property, spreadability on skin and solubilizing property as a base for transdermal administration, but also properties required for pharmaceutical preparation for transdermal administration. Therefore the base can be used, as it is, as a base for a pharmaceutical preparation such as emulsion lotion, patch, emulsion, solution or aerosol.

When the base is used for the other pharmaceutical preparations for transdermal administration such as ointment, cream, liniment, plaster-mass, plaster, tape, pack and suspension, an optional inert ingredient required for each preparation can be added to the base of the present invention.

As the optional inert ingredient, for instance, a cream-forming agent is used in a cream. The cream-forming agent includes o/w type agent (so-called as "cream") wherein a component such as lanolin, propylene glycol, stearyl alcohol, vaseline, silicone oil, liquid paraffin and/or glyceryl monostearate is emulsified or dispersed in a water phase with or without a surfactant and w/o type agent which is prepared by adding water to a component such as vaseline, a higher aliphatic alcohol and/or liquid parrafin followed by emulsifying or dispersing with a nonionic surfactant having little hydrophilic group.

When the base is used for a suspension, there can be added as the optional inert ingredient a suspending agent prepared by gelatinizing a mixture of water and starch, glycerin, high-viscosity carboxymethylcellulose or carboxyvinylpolymer. When the base is used for a suspending lotion, there can be added as the optional inert ingredient an additive prepared by mixing water and a gum such as sodium alginate, gum arabic, pectin or tragacanth gum, a cellulose compound such as methylcellulose or hydroxyethylcellulose or a clay such as bentonite or Veegum HV. When the base is used for a liniment, there can be added as the optional inert ingredient a vegetable oil such as olive oil, sesame oil, almond oil, cotton seed oil or turpentine oil. When the base is used for a cataplasm, there can be added as the optional inert ingredient a water-soluble polymer such as polyacrylic acid or a salt thereof, a polyvinyl alcohol or polyvinylpyrrolidone; or a crosslinked water-soluble polymer which is crosslinked with a salt of polyvalent metal such as alum or crosslinked by a physical treatment such as radiation exposure.

A flavoring agent as well as a preservative such as paraoxybenzoic acid, methylparaben, ethylparaben, propylparaben, chlorobutanol or benzylalcohol may be also added to the base of the present invention. Further, there can be added various kind of emulsifying agent, dispersing agent, moistening agent, stabilizer and/or antiseptic, as well as pH adjusting agent according to the use of the base. Although the pH adjusting agent is not particularly limited if it is usable in the field of pharmaceutical preparation, there can be exemplified an inorganic acid such as hydrochloric acid, sulfuric acid, nitric acid, hydrobromic acid or phosphoric acid; an organic acid such as acetic acid, succinic acid, fumaric acid, malic acid, oxalic acid, lactic acid, glutaric acid, salicylic acid or tartaric acid; and a salt thereof.

The base of the present invention can be prepared according to the usual method, for example, easily prepared by mixing a fatty acid ester, an alcohol, water and if necessary, optional inert ingredient. The base of the present invention can be also prepared, for example, by a method wherein a fatty acid ester is dissolved in an alcohol, then water is added thereto followed by dissolving, emulsifying or dispersing to prepare a base, and then, if necessary, optional inert ingredient is added thereto followed by kneading, emulsifying or suspending. On keading, emulsifying or suspending, there can be used any mixing machine which is usually usable in the field of pharmaceutical preparation, for example, screw mixer, homomixer, kneader, roller mill or the like.

Another aspect of the present invention is a pharmaceutical preparation for transdermal administration, which enables a medicinal compound to be well absorbed transdermally. The pharmaceutical preparation of the present invention contains the medicinal compound in the above-mentioned base of the present invention.

The medicinal compound usable in the pharmaceutical preparation of the present invention is not particularly limited and any compound can be used. Medicinal compounds having systemic action or local action which can be administered transdermally may be usually used.

Representative examples of such medicinal compounds are, for instance, as follows:

Agents affecting cardiovascular system antihypertensives, e.g. Ca antagonist such as clentiazem (chemical name: (+)-(2S,3S)-3-acetoxy-8-chloro-5-(2-dimethylaminoethyl)-2,3-dihydro-2-(4-methoxyphenyl)-1,5-benzothiazepin-4 -(5H)-one), diltiazem or nifedipine, ACE inhibitor such as imidapril, enalapril or captopril, adreno-β-receptor blocker such as bisoprolol fumarate, denopamine, propranolol or isoproterenol hydrochloride and adreno-α 2-receptor agonist such as clonidine; coronary vasodilator such as nitroglycerin, isosorbide dinitrate or molsidomine; cardiac glycoside such as digoxin; cerebral vasodilator such as nicergoline, and the like.

Neurotropic agent diazepam, imipramine, and the like.

Agents affecting central nervous system agent affecting autonomic nervous system such as dl-methylephedrine hydrochloride; antimotionsickness agent such as diphenhydramine; antipyretic analgesic such as salicylic acid; cholinesterase inhibitor such as physostigmine, and the like.

Agents affecting respiratory organs bronchodilator such as 8-hydroxy-5-[(1R)-1-hydroxy-2-[N-(1R)-2-(p-methoxyphenyl)-1-methylethyl)amino]ethyl]-carbostyril or epinephrine, and the like.

Agents affecting digestive organs enterokinesis improving agent such as trimebutine malealte, and the like.

Agents affecting endocrine system or metabolism vitamin preparation such as vitamin A, vitamin D, vitamin E or vitamin K; polypeptide hormone such as LH-RH, TRH, calcitonin or ANP; endogenous opioid peptide such as endorphin or dynorphin; androgen such as testosterone; estrogen such as estradiol; adrenocortical steroid such as corticosteroid; and the like.

Antineoplastic agent 5-fluorouracil, 6-mercaptopurine and the like.

Non-steroidal anti-inflammatory drug aspirin, diclofenac, ibuprofen, indometacin, ketoprofen and the like.

These medicinal compound may be usable in their free form or in a form of pharmacologically acceptable salt thereof. Representative examples of such salt are, for instance, a salt with an inorganic acid such as hydrochloric acid, sulfuric acid or nitric acid, a salt with an organic acid such as fumaric acid, maleic acid or acetic acid, and the like.

Although, as above-mentioned, almost all of medicinal compounds can be used in the pharmaceutical preparation for transdermal administration of the present invention, there are preferably used lipophilic medicinal compounds for example, compounds having a partition coefficient between water and octanol of at most about $10^9$, preferably about $10^{-7}$ to about $10^7$, more preferably about $10^{-4}$ to about $10^6$. When a polyhydric alcohol is used as a component of the base, there are preferably used medicinal compounds having a partition coefficient between water and octanol of about $10^2$ to about $10^6$.

The amount of a medicinal compound to be used is not particularly limit in the pharmaceutical preparation for transdermal administration of the present invention and may be suitably determined according to pharmacological action of the medicinal compound to be used and disease, age, sympton and weight of a patient. It is preferable from the viewpoint of characteristics of pharmaceutical preparation for transdermal administration that the pharmaceutical preparation contains a medicinal compound usually in an amount of about 0.01 % to about 20 % (w/w or w/v) on the basis of the total amount of the pharmaceutical preparation.

When there is used a medicinal compound such as vitamin D or a hormone, which can bring about effectiveness in a very little dose, the above-mentioned amount of the medicinal compound may be further decreased.

Typical example of the pharmaceutical preparation for transdermal administration of the present invention will be explained in the followings, with giving an example of a pharmaceutical preparation containing as a medicinal compound a lipophilic vitamin e.g. biologically active vitamin $D_3$ such as alphacalcidol or calcitriol. In such pharmaceutical preparation, among the above-mentioned bases of the present invention there are preferably used a base comprising as the fatty acid ester a) an ester of a fatty acid having 1 to 32 carbon atoms and an aliphatic monohydric alcohol having 1 to 30 carbon atoms or b) an ester of a saturated or unsaturated fatty acid having 10 to 22 carbon atoms and a mono- or polyglycerin, as the alcohol a monohydric or polyhydric alcohol having at most 5 carbon atoms and water, and the like.

Among these there are more preferable a base comprising isopropyl myristate, ethanol and water, a base comprising isopropyl caprate, ethanol and water, and the like.

In a preferable embodiment of the above-mentioned pharmaceutical preparation, a medicinal compound, for example, biologically active vitamin $D_3$ is contained in a base comprising 1 to 50 % by volume of the fatty acid ester, 10 to 90 % by volume of the alcohol and 5 to 80 % by volume of water respectively on the basis of the total amount of the three components.

The above-mentioned phamaceutical preparation for transdermal administration containing biologically active vitamin $D_3$ enables the medicinal compound to be well absorbed transdermally. Moreover, the above-mentioned pharmaceutical preparation enables an effective plasma concentration of biologically active vitamin $D_3$, whose half-life in vivo is short, to be stably maintained for a long time in a small dose, contrary to the case orally administering biologically active vitamin $D_3$. In case of administrating the phamaceutical preparation containing biologically active vitamin $D_3$ of the present invention, there can be therefore decreased a side effect such as hypercalcemia which often occurs in case orally administering biologically active vitamin $D_3$.

Furthermore, a medicinal compound having a short half-life in vivo, such as biologically active vitamin $D_3$, is stable for a long period in the pharmaceutical preparation of the present invention wherein the compound is contained in the above-mentioned base of the present invention.

The pharmaceutical preparation for transdermal administration of the present invention may be various form of formulation such as emulsion lotion, patch, lotion, solution, aerosol, ointment, cream, liniment, tape, pack or suspension.

A pharmaceutical preparation in a desired form of formulation can be prepared easily by adding additional components required for the formulation, as above-mentioned. For example, an emulsion lotion can be prepared by mixing a medicinal compound and the base of the present invention with stirring, then, if necessary, adding additives such as colorant and flavoring agent followed by mixing, and filtrating the resulting mixture. A patch can be prepared by mixing a medicinal compound and the base and then impregnating a backing film with the obtained mixture. A liniment can be prepared by dissolving a medicinal compound in the base and then mixing the obtained mixture. A cataplasm can be prepared by kneading a medicinal compound, the base and desired additives and then spreading the obtained mixture on a backing film.

The present invention is more specifically described and explained by means of the following Experimental Examples and Examples. It is to be understood that the present invention is not limited to the Examples, and various changes and modifications may be made in the invention without departing from the spirit and scope thereof.

Experimental Example 1

An abdominal skin was taken from a Wistar male rat weighing 250 to 300 g which was previously shaved with an electric hair clippers on the day before. The skin was placed in a standard Franz-type diffusion cell (trade name: FDC-400, CROWN GLASS COMPANY (U.S.A.)). Constant temperature (37°C) water was circulated in a jacket part of the cell and saline solution was filled in a receptor compartment of the cell.

To a base for transdermal administration having each composition shown in Table 1, was added 8-hydroxy-5-[(IR)-1-hydroxy-2-[N-(1R)-2-(p-methoxyphenyl)-1-methylethyl)amino]ethyl]carbostyril hydrochloride, so as to prepare an 1 % (w/v) solution thereof. To the donor compartment of the cell was administered 0.8 ml of the obtained solution. The concentrations of the medicinal compound in the receptor compartment were measured at predetermined intervals and cumulative amounts of the medicinal compound up to 8 hours after the administration were calculated.

The results are shown in Table 1.

Table 1

| Pharmaceutical preparation | Composition of base (v/v %) | | | Cumulative amount of medicinal compound ($\mu$ g/cm$^2$) |
|---|---|---|---|---|
| | Water | Fatty acid ester | Alcohol | |
| Present invention | | | | |
| 1 | 10.7 | IPC 17.9 | Ethyl alcohol 71.4 | 1194 |
| 2 | 7.4 | IPP 18.5 | Ethyl alcohol 74.1 | 1073 |
| 3 | 7.4 | IPM 18.5 | Butyl alcohol 74.1 | 2340 |
| 4 | 11.5 | IPM 11.5 | Ethyl alcohol 77.0 | 1154 |
| Reference | | | | |
| 5 | | IPM 100 | | 84 |
| 6 | | | Ethyl alcohol 100 | 2 |
| 7 | 100 | | | 24 |
| 8 | | IPM 50 | Ethyl alcohol 50 | 530 |
| 9 | 50 | IPM 50 | | 31 |
| 10 | 50 | | Ethyl alcohol 50 | 9 |
| 11 | | IPP 50 | Ethyl alcohol 50 | 498 |
| 12 | | IPC 50 | Ethyl alcohol 50 | 375 |

Note: IPC: Isopropyl caprate
IPM: Isopropyl myristate
IPP: Isopropyl palmitate

Experimental Example 2

To a base for transdermal administration having each composition shown in Table 2, clentiazem maleate was added as a medicinal compound so as to prepare an 1 % (w/v) solution or suspension thereof.

The procedure of Experimental Example 1 was repeated except for using lml of the obtained mixture.

Cumulative amounts of the medicinal compound penetrated up to 8 hours after the administration were obtained in the same manner as in Experimental Example 1.

The results are shown in Table 2.

Table 2

| Pharmaceutical preparation | | Composition of base (v/v %) | | | Cumulative amount of medicinal compound ($\mu$ g/cm²) |
|---|---|---|---|---|---|
| | | Water | Fatty acid ester | Alcohol | |
| Present invention | 1 | 10.7 | IPM 17.9 | Ethyl alcohol 71.4 | 2062 |
| | 2 | 21.9 | IPM 15.6 | IPA 62.5 | 2515 |
| Reference | 3 | | IPM 100 | | 221 |
| | 4 | | | Ethyl alcohol 100 | 38 |

Note: IPM: Isopropyl myristate
IPA: Isopropyl alcohol

Experimental Example 3

To a base for transdermal administration having each composition shown in Table 3, 17β-estradiol was added as a medicinal compound so as to prepare a 0.1 % (w/v) solution or suspension thereof.

The procedure of Experimental Example 1 was repeated except for using lml of the obtained mixture.

Cumulative amounts of the medicinal compound penetrated up to 8 hours after the administration were obtained in the same manner as in Experimental Example 1.

The results are shown in Table 3.

Table 3

| Pharmaceutical preparation | Composition of base (v/v %) | | | Cumulative amount of medicinal compound ($\mu$ g/cm$^2$) |
|---|---|---|---|---|
| | Water | Fatty acid ester | Alcohol | |
| **Present invention** | | | | |
| 1 | 10.7 | IPM 17.9 | Ethyl alcohol 71.4 | 9.7 |
| 2 | 15 | IPM 15 | Propylene glycol 70 | 5.1 |
| **Reference** | | | | |
| 3 | | IPM 100 | | 0.18 |
| 4 | 50 | | Ethyl alcohol 50 | 0.30 |
| 5 | | | Ethyl alcohol 100 | 0.15 |
| 6 | | | Propylene glycol 100 | 0.03 |
| 7 | | IPM 50 | Propylene glycol 50 | 0.38 |
| 8 | 100 | | | 0.05 |

Note: IPM: Isopropyl myristate

Experimental Example 4

The procedure of Experimental Example 1 was repeated except that 17β-estradiol as a medicinal compound was added to a base for transdermal administration having each composition shown in Table 4, so as to prepare a 0.1 % (w/v) solution thereof and saline solution containing 20 % (v/v) of polyethylene glycol 400 was used as a receptor fluid.

Cumulative amounts of the medicinal compound penetrated up to 8 hours after the administration were obtained in the same manner as Experimental Example 1.

The results are shown in Table 4.

Experimental Example 5

To a base for transdermal administration having each composition shown in Table 5, antipyrine as a med-

## Table 4

| Pharmaceutical preparation | | Composition of base (v/v %) | | | | Cumulative amount of medicinal compound ($\mu$ g/cm²) | |
|---|---|---|---|---|---|---|---|
| | | Water | Fatty acid ester | | Alcohol | | |
| Present invention | 1 | 4 | ISIS | 16 | Isopropyl alcohol | 80 | 11.2 |
| | 2 | 6.8 | BS | 16.9 | Ethyl alcohol | 76.3 | 41.4 |
| | 3 | 2.9 | ODM | 19.4 | Isopropyl alcohol | 77.7 | 16.1 |
| | 4 | 17 | GMO | 28 | Ethyl alcohol | 55 | 11.5 |
| | 5 | 50 | IPM | 5 | Ethyl alcohol | 45 | 53.2 |
| Reference | 6 | | | | Isopropyl alcohol | 100 | 1.2 |
| | 7 | | | | Ethyl alcohol | 100 | 0.8 |

Note:  ISIS: Isocetyl isostearate
BS: Butyl stearate
ODM: Octyldodecyl myristate
GMO: Glyceryl monooleate
IPM: Isopropyl myristate

icinal compound was added so as to prepare an 1 % (w/v) solution thereof.

The procedure of Experimental Example 1 was repeated except for using 1ml of the obtained mixture.

Cumulative amounts of medicinal compound penetrated (Y axis) were plotted against time (X axis). A slope of a part of the straight line was regarded as a flux of the medicinal compound and a X-intercept was regarded as a lag-time. These values were compared between some preparations as shown in Table 5.

Table 5

| Pharmaceutical preparation | Composition of base (v/v %) | | | | | lag time (hour) | Flux ($\mu$ g/cm²/hour) |
|---|---|---|---|---|---|---|---|
| | Water | Fatty acid ester | | Alcohol | | | |
| **Present invention** | | | | | | | |
| 1 | 10.7 | IPM | 17.9 | Ethyl alcohol | 71.4 | 0.6 | 63.6 |
| **Reference** | | | | | | | |
| 2 | 100 | | | | | 2.8 | 0.45 |
| 3 | | | | Ethyl alcohol | 100 | 5.1 | 14.3 |
| 4 | 50 | | | Ethyl alcohol | 50 | 4.5 | 4.4 |
| 5 | | IPM | 20 | Ethyl alcohol | 80 | 1.7 | 54.3 |

Note:  IPM:  Isopropyl myristate

EP 0 581 587 A2

Experimental Example 6

Object:

A vitamin D deficient rat was administered with a pharmaceutical preparation for transdermal administration of the present invention wherein bioligically active vitamin $D_3$ was contained in a base consisting of isopropyl myristate, ethanol and water, in order to examine the action of the pharmaceutical preparation on plasma concentration of calcium and bone mineral density.

Preparation of pharmaceutical preparation:

Biologically active vitamin $D_3$ was dissolved in a base of isopropyl myristate, ethanol and water (proportion of the components = 1:4:0.6) to give a 0.01 μg/ml solution of biologically active vitamin $D_3$. A patch (2.5 cm²) impregnated with 1 ml of the obtained solution was prepared as a pharmaceutical preparation for transdermal administration of the present invention. As a control, a patch impregnated with 1ml of only the above-mentioned base was prepared.

Test method:

Wistar male rats (3.5 weeks) were fed with a diet which contains 0.44 % of calcium and no vitamin D (a diet for experiment, trade name: Diet 11, commercially available from Clea Japan Inc. ) for 2 weeks and then fed with a diet which contains little calcium and no vitamin D prepared by removing calcium from Diet 11, for a week. After the feeding, the rats were divided into two groups. While breeding with Diet 11, one group (medicinal compound-administered group) was administered with the patch containing biologically active vitamin $D_3$ and the other group (control group) was administered with the patch impregnated with only the above-mentioned base, each patch being attached to shaved abdomen or regiones dorsales of each rat. Each patch was attached with exchanging the newly prepared patch twice a week for 7 weeks in both groups.

Blood was collected from jugular vein of rat at weekly internals after the administration and the concentration of calcium in plasma was measured by atomic absorption spectrophotometry.

Femur was extracted from each rat 7 weeks after the administration and dried under reduced pressure at 100°C for 8 hours. Then the bone mineral density of the femur was calculated.

The bone mineral density was obtained by measuring a bone mineral content (BMC) by means of single photon absorptiometry with a bone mineral analyzer (trade name: DCS-600, commercially available from Aloka Co., Ltd.), measureing an area of the bone (AREA) projected by radiography and then calculating BMC/AREA.

Result:

As shown in Table 6, the biologically active vitamin $D_3$ was absorbed transdermally. Therefore the absorption of calcium in gut was improved and the concentration of calcium in plasma was restored to almost the normal value.

On the other hand, the concentration of calcium in the control group further lowerd compared with its initial value, and the values at 3 weeks to 7 weeks after the administration were almost one-second of that of normal group which was fed with normal solid diet (trade name: CRF-1, commercially available from Charles River Japan Inc.).

As shown in Table 7, the bone mineral density of the vitamin D deficient rat was statistically high in the medicinal compound-administered group compared with that in the control group.

As shown in Fig. 1, when the femur was divided in equal 5 parts perpendicularly to its longitudinal direction, the bone mineral density at every part was high in the medicinal compound-administered group compared with that in the control group.

Table 6

| Time after | Concentrations of calcium in plasma (mg/dl) | | |
|---|---|---|---|
| administration (week) | Control group | Medicinal compound-administered group | Normal group |
| 0 | 8.0 | 7.6 | 9.6 |
| 1 | 8.0 | 8.0 | 9.4 |
| 2 | 7.4 | 9.2 | 9.7 |
| 3 | 5.4 | 8.6 | 9.7 |
| 4 | 5.3 | 9.5 | 9.3 |
| 5 | 5.0 | 9.4 | 10.3 |
| 6 | 4.8 | 9.2 | 9.7 |
| 7 | 4.9 | 8.2 | 9.3 |

Table 7

| | Dry weight of rat femur (mg) | BMC (mg) | AREA (cm²) | Bone mineral density(mg/cm²) |
|---|---|---|---|---|
| Medicinal compound-administered group | 457.6 ±11.0 | 196.4 ±7.73 | 1.942 ±0.039 | 101.0 # ±2.56 |
| Control group | 382.7 ±19.4 | 147.4 ±7.59 | 1.719 ±0.058 | 85.6 ±2.11 |

#:  $p < 0.05$ vs control

Experimental Example 7

Object:

A normal rat was administered with a pharmaceutical preparation for transdermal administration of the present invention wherein biologically active vitamin $D_3$ was contained in a base consisting of isopropyl myristate, ethanol and water, in order to examine the bioavailability.

Preparation of pharmaceutical preparation:

Biologically acitive vitamin $D_3$ was dissolved in a base of isopropyl myristate, ethanol and water (proportion of the components = 1:4:0.6 or 1:9.5:9.5) to give a 0.1 μg/ml solution of biologically active vitamin $D_3$. A patch

(5cm²) impregnated with lml of the obtained solution was prepared as a pharmaceutical preparation for trasdermal administration of the present invention. As a reference, a patch impregnated with 1 ml of an 1 μg/ml solution of biologically active vitamin $D_3$ in ethanol was prepared.

Test method:

Each patch was attached to an abdomen of rat which was previously shaved the day before.

Blood was collected from jugular vein of the rat 6, 24 and 48 hours after the attaching. Plasma concentrations of biologically active vitamin $D_3$ (1α, 25(OH)$_2$D$_3$) were measured by HPLC-RRA method and the obtained bioavailabilities were compared between groups.

Result:

As shown in Table 8, the plasma concentration of 400 to 500 pg/ml was maintained for 24 to 48 hours after the administration in the group administered with the pharmaceutical preparation for transdermal administration of the present invention. On the other hand, in case of administering the reference pharmaceutical preparation, even ten times dose (1 μg) could not raise the plasma concentration from the concentration before the administration.

## Table 8

| Composition of base | Dose ($\mu$ g/rat) | Plasma concentration (pg/ml) | | | |
|---|---|---|---|---|---|
| | | 0hour | 6hours | 24hours | 48hours |
| IPM:E:W (1:4:0.6) | 0.1 | 117 | 212 | 405 | 436 |
| IPM:E:W (1:9.5:9.5) | 0.1 | 117 | 336 | 564 | 516 |
| Ethanol alone | 1 | 101 | 144 | 226 | 118 |

IPM: Isopropyl myristate
E : Ethanol
W : Water

Example 1

Into a base consisting of 17.9 % (v/v) of isopropyl myristate, 71.4 % (v/v) of ethyl alcohol and 10.7 % (v/v) of water, 17β-estradiol was dissolved so that the concentration thereof became 0.1 % (w/v). Hydroxypropylcellulose-SL (commercially available from Nippon Soda Co., Ltd) was added to thus obtained pharmaceutical preparation in an amount of 200 mg per 1 g of the pharmaceutical preparation and dissolved therein. The obtained mixture was then spread on a polyethylene sheet to form a 1 cm radius circle, thereby giving a gel ointment.

Using the obtained gel ointment, the procedure of Experimental Example 1 was repeated except that saline solution containing 20 % (v/v) of polyethylene glycol 400 was used as a receptor fluid, to examine a skin permeability of the medicinal compound. The cumulative penetration amount of the medicinal compound penetrated up to 24 hours was found to be 41.7 (μg/cm²). On the other hand in case of using a pharmaceutical preparation for transdermal administration prepared by adding hydroxypropylcellulose-SL to a 0.1 % (w/v) solution of 17β-estradiol dissolved in ethanol in the same way as described above, the cumulative amount of the medicinal compound penetrated up to 24 hours was found to be 3.0 (μg/cm²).

## Example 2

To 8 g of vaseline was added 0.1 g of a surfactant (trade name: Rheodol SP-010, commercially available from Kao Corporation) and the obtained mixture was heated to 70° to 80°C to give a solution. After cooling the solution to 50° to 60°C, thereto was gradually added with stirring 2 g of a mixture which was prepared by adding 17$\beta$-estradiol to a base consisting of 17.9 % (v/v) of isopropyl myristate, 71.4 % (v/v) of ethyl alcohol and 10.7 % (v/v) of water so that the concentration of 17$\beta$-estradiol in the solution became 0.1 % (w/v). Then the resultant solution was cooled with stirring to give an ointment.

The cumulative amount of the medicinal compound penetrated up to 24 hours was examined in the same manner as in Example 1 and found to be 12.4 ($\mu$g/cm$^2$). On the other hand, in case of using a pharmaceutical preparation for transdermal administration preparared by adding a 0.1 % (w/v) solution of 17$\beta$-estradiol dissolved in ethyl alcohol to a vaseline containing Rheodol SP-010 in the same manner as described above, the cumulative amount of the medicinal compound penetrated up to 24 hours was found to be 6.8 ($\mu$g/cm$^2$).

## Example 3

Into a base consisting of 17.9 % (v/v) of isopropyl myristate, 71.4 % (v/v) of ethyl alcohol and 10.7 % (v/v) of water, was dissolved biologically active vitamin D$_3$ (calcitriol, commercially available from Wako Pure Chemical Industries Ltd.) so that the concentration thereof became 0.05 % (w/v). The procedure of Experimental Example 1 was repeated except for using lml of thus obtained pharmaceutical preparation for transdermal administration and using saline solution containing 10 % (v/v) of polyethylene glycol 400 and 5 % (v/v) of ethanol as a receptor fluid, to examine the cumulative amount of the medicinal compound penetrated up to 8 hours after the administration was found to be 2.1 $\mu$g/cm$^2$.

On the other hand, in case of using a phamaceutical preparation for transdermal administration of a 0.05 % (w/v) solution of biologically active vitamin D$_3$ dissolved in ethyl alcohol and in case of using a pharmaceutical preparation for transdermal administration wherein 0.05 % (w/v) of biologically active vitamin D$_3$ is contained in a vehicle consisting of ethanol or a vehicle consisting of 97 % (v/v) of propylene glycol and 3 % (v/v) of Azone, the cumulative amounts of the medicinal compound penetration up to 8 hours after the administration were found to be 0.1 ($\mu$g/cm$^2$) in both cases.

## Example 4

Into 30 ml of a base consisting of 17.9 % (v/v) of isopropyl myristate, 71.4 % (v/v) of ethyl alcohol and 10.7 % (v/v) of water, was dissolved 0.05 g of imidapril. A circular non-woven fablic (trade name: ED-4150, commercially available from Japan Vilene Co., Ltd) having a diameter of 2.5 cm was impregnated with 0.3 ml of the obtained solution, then laminated with a circular alminium foil having a diameter of 3 cm thereon and further covered with a circular poly(vinyl chloride) film having a diameter of 4 cm which surface was coated with an pressure-sensitive adhesive, to prepare a patch.

The obtained patch (dose: 2 mg/kg) was attached to the abdomen of a Wistar male rat weighting about 250 to about 300 g which was previously shaved on the day before, and fixed with an elastic bandage. Blood was collected from jugular vein of the rat at predetermined intervals and the concentration of a metabolite (chemical name: (4S)-1-methyl-3-[(2S)-2-[N-(1S)-1-carboxy-3-phenylpropyl)amino-propionyl]-2-oxoimidazolidine-4-carboxylic acid) in plasma was measured by means of radioimmunoassay.

The results are shown in Table 9.

Table 9

| | Plasma concentration(ng/ml) | | | |
|---|---|---|---|---|
| Time (hour) | 1 | 2 | 4 | 6 |
| Pharmaceutical preparation of the present invention | 8.9 | 99.3 | 257.9 | 174.5 |

In case of using a patch prepared in the same manner as discribed above except that imidapril was dissolved in water, the concentration of the active metabolite in plasma was less than the detection limit.

Example 5

Into 100 ml of a base consisting of 17.9 % (v/v) of isopropyl myristate, 71.4 % (v/v) of ethyl alcohol and 10.7 % (v/v) of water, was dissolved 1 mg of biologically active vitamin $D_3$ (calcitriol).

Using 1 ml of thus obtained solution, a patch was prepared in the same manner as in Example 4 and administered transdermally to Wister male rats. Blood was collected from abdominal aorta and the concentration of the biologically active vitamin $D_3$ in blood was measured by HPLC at 24 hours and 48 hours after administration respectively.

As a reference, a patch was prepared in the same manner as described above except that 1 mg of biologically active vitamin $D_3$ was dissolved in 100 ml of ethyl alcohol, and using thus obtained reference patch, the concentration of biologically active vitamin $D_3$ in blood was also measured by HPLC.

The results are shown in Table 10.

Table 10

|  | Plasma concentration(ng/ml) | |
| --- | --- | --- |
| Time (hour) | 24 | 48 |
| Pharmaceutical preparation of the present invention | 9.8 | 10.1 |
| Reference | less than detection limit | less than detection limit |

Example 6

Epicutaneous testing patch (trade name, Finn chamber, commercially available from Taisho Pharmaceutical Co., Ltd) wherein each base for transdermal administration shown in Table 11 was impregnated was attached to brachial skin of a healthy person. The patch was detached from the skin 7 hours after the attaching and the condition of the skin was observed 24 hours after the attaching. The estimation was carried out based on the following criterion.

The results are shown in Table 11.

EP 0 581 587 A2

Table 11

| Pharmaceutical preparation | Composition of base (v/v %) | | | | | Estimation |
|---|---|---|---|---|---|---|
| | Water | Fatty acid ester | | Alcohol | | |
| Present invention | | | | | | |
| 1 | 10.7 | IPM | 17.9 | Ethyl alcohol | 71.4 | ± |
| 2 | 21.9 | IPM | 15.6 | Isopropyl alcohol | 62.5 | ± |
| Reference | | | | | | |
| 3 | – | Azone | 3 | Propylene glycol | 97 | +2 |
| 4 | 70 | Menthol | 10 | Ethyl alcohol | 20 | +2 |

Note:  IPM:  Isopropyl myristate
  Criterion:
    ±:  no change
    +:  slight erythema
    +2:  clear erythema
    +3:  servere erythema and bleb

Example 7

A mixture of 6 g of stearyl alcohol, 1 g of polyethylene glycol 6000 and 1 g of 1,2,6-hexanetriol was heated to 80° to 85°C to prepare a solution. 17β-estradiol was added thereto and mixed therein so that the final con-

22

centration thereof became 0.1 % (w/w). Thereto was added a base of 8.4 g of 1,3-butanediol, 1.8 g of water, 1.8 g of isocetyl isostearate and 0.1 g of Rheodol SP-010, which base was separately heated at 80°C, and the resulting mixture was stirred and then cooled with mixing till the mixture solidificated. Thus an ointment was prepared.

Example 8

The procedure of Example 7 was repeated except that 1.8 g of isopropyl capronate was used instead of 1.8 g of isocetyl isostearate, to give an ointment.

Example 9

Into a mixture of 20 g of ethanol and 30 g of water was dissolved 1 g of antipyrine. To the obtained solution was added 10 g of polyvinyl alcohol (completely saponificated PVA, degree of polymerization: 2000) and the obtained mixture was heated with stirring to prepare a solution. Thereto were added 20 g of glycerin and 10 g of hexaglyceryl monolaurate, followed by stirring. Water was added to the obtained mixture to make the total weight 100 g, followed by sufficiently stirring. Then the mixture was freezed and thawed to prepare a polyvinyl alcohol gel.

Example 10

In a beaker were sufficiently stirred 0.5 g of carboxyvinyl polymer (trade name: HIVISWAKO 104, commercially available from Wako Pure Chemical industries, Ltd.), 7.5 g of isopropyl alcohol and 10 g of water. 17β-estradiol was added thereto so that the final concentration thereof bacame 0.1 % (w/w) and then a solution of 2.5 g of isopropyl alcohol and 0.5 g of butyl stearate was gradually added. With stirring the obtained mixture, 0.25 g of diisopropanolamine was gradually added thereto and the obtained mixture was stirred to give a gel having a high viscosity.

Using 1 g of thus obtained gel, the accumulative penetration amount of the medicinal compound penetrated up to 8 hours after the administration was measured in the same manner as in Example 1 and found to be 15.5 $\mu$g/cm$^2$. On the other hand, in case of using a gel prepared by adding a 0.1 % (w/w) solution of 17β-estradiol dissolved in isopropyl alcohol to a solution of carboxyvinylpolymer (trade name: HIVISWAKO 104) dissolved in isopropyl alcohol, the cumulative penetration amount of the medicinal compound penetrated up to 8 hours after the administration was found to be 0.89 $\mu$g/cm$^2$.

## Claims

1.  A base for transdermal administration for a medicinal compound comprising a fatty acid ester, an alcohol and water.

2.  A base as claimed in claim 1, wherein the fatty acid ester is a higher fatty acid ester and the alcohol is a monhydric alcohol or a polyhydric alcohol.

3.  A base as claimed in claim 1 or claim 2, wherein the base comprises about 0.1 to about 50% by volume of the fatty acid ester, about 10 to about 90% by volume of the alcohol and about 1 to about 80% by volume of water based on the total volume of these three components.

4.  A base as claimed in any one of the preceding claims, wherein the alcohol is an alcohol having 2 to 12 carbon atoms, and the fatty acid ester is (A) an ester of a fatty acid having 1 to 32 carbon atoms and an aliphatic monohydric alcohol having 1 to 30 carbon atoms or (B) an ester of a saturated or unsaturated fatty acid having 10 to 22 carbon atoms and a mono- or polyglycerin.

5.  A base as claimed in any one of the preceding claims, wherein the alcohol is a monhydric, dihydric or trihydric alcohol having 2 to 4 carbon atoms, and the fatty acid ester is an ester of a fatty acid having 6 to 18 carbon atoms and an aliphatic monhydric alcohol having 3 to 20 carbon atoms or an ester of a saturated or unsaturated fatty acid having 12 to 18 carbon atoms and a mono- or polyglycerin selected from the group consisting of monoglycerin, diglycerin, triglycerin, tetraglycerin, pentaglycerin and hexaglycerin.

6. A base as claimed in claim 1 or claim 2, wherein the fatty acid is isopropyl myristate or ispropyl caprate, and the alcohol is ethanol.

7. A pharmaceutical preparation for transdermal administration comprising a medicinal compound and a base for transdermal administration comprising fatty acid ester, an alcohol and water as claimed in anyone of the preceding claims.

8. A pharmaceutical preparation as claimed in claim 7, wherein the fatty acid ester is isopropyl myristate or isopropyl caprate and the alcohol is ethanol.

9. A pharmaceutical preparation as claimed in claim 7 or claim 8, wherein the medicinal compound is a lipophilic compound.

10. A pharmaceutical preparation as claimed in claim 8, wherein the medicinal compound is a biologically active vitamin $D_3$.

# FIG. 1